# EUROPEAN PATENT APPLICATION

(11) **EP 3 315 059 A1**
(43) Date of publication of application: **02.05.2018**
(21) Application number: 16817731.9
(22) Date of filing: 15.06.2016
(51) Int. Cl.: A61B 1/06, A61B 1/00, G02B 23/24

(54) **ENDOSCOPE SYSTEM**

(30) Priority: 29.06.2015 JP 2015130243
(71) Applicant: Olympus Corporation, Hachioji-shi Tokyo 192-8507 (JP)
(72) Inventor: KURIHARA, Kozue, Hachioji-shi Tokyo 192-8507 (JP); OGAWA, Tomoaki, Hachioji-shi Tokyo 192-8507 (JP)
(74) Representative: Schicker, Silvia
(86) International application number: PCT/JP2016/067783
(87) International publication number: WO 2017/002622

(57) **Abstract**

An endoscope system according to the present invention is an endoscope system including an endoscope including an inserted portion and a connector portion, and a water-resistant cap configured to be airtightly installed at the connector portion. The connector portion includes a base portion, a sleeve portion, and an airtightness release portion provided at an end portion of the sleeve portion and configured to release an airtight state between the connector portion and the water-resistant cap. The water-resistant cap includes a cap main body and an abutting portion configured to abut on the sleeve portion. When the water-resistant cap is installed at the connector portion, the endoscope system takes any state of a first state where the abutting portion abuts on the sleeve portion at a side of the base portion with respect to the airtightness release portion to hold an airtightness between the water-resistant cap and the connector portion, and a second state where the abutting portion abuts on or faces the sleeve portion including the airtightness release portion to become non-airtight between the water-resistant cap and the connector portion.

## Description

### Field

The present invention relates to an endoscope system.

### Background

Conventionally, in the field of medicine, an endoscope system is employed when an inside of a subject such as a patient is observed. The endoscope system includes an endoscope, for example, where an image sensor is provided at a distal end and includes an inserted portion to be inserted into a body cavity of the subject, and an image processing device, which is coupled to the endoscope via a cable and performs image processing of an in-vivo image corresponding to an imaging signal generated by the image sensor to display the in-vivo image on a display unit or the like.

On the endoscope, which the cable is removed before and after the observation inside the subject, a sterilization treatment by, what is called, an autoclave or the like and a disinfectant treatment by an antiseptic solution are performed. In this respect, such that liquid such as water vapor and the antiseptic solution does not internally enter from an electric connector of the endoscope, a water-resistant cap is installed at an electric connector portion of the endoscope (for example, see Patent Literature 1). When an elastic member, such as an O-ring, provided on a wall surface of the water-resistant cap is interposed between the electric connector portion of the endoscope and the water-resistant cap, for example, the electric connector portion of the endoscope and the water-resistant cap have a mutually airtight state. Citation List

### Patent Literature

Patent Literature 1: JP 5-300870 A

### Summary

### Technical Problem

Now, in the sterilization by the autoclave or the like, negative pressure processing is performed before a sterilization process, and then, the sterilization process is performed under a positive pressure environment. After the sterilization, when the water-resistant cap is attempted to be removed from the electric connector portion of the endoscope whose inside is in a negative pressure state, since the O-ring sticks to the wall surface of the endoscope, there is a problem that the water-resistant cap cannot be removed from the electric connector portion of the endoscope.

The present invention has been made in view of the above-described problem. An object of the present invention is to provide an endoscope system that ensures easily removing a water-resistant cap from an electric connector portion of an endoscope whose inside is in a negative pressure state.

### Solution to Problem

To solve the problem described above and to achieve the object, an endoscope system according to the present invention includes: an endoscope including an inserted portion to be inserted into an inside of a subject and a connector portion for electrically coupling to an external connector, the endoscope being configured to observe the inside of the subject; and a water-resistant cap configured to be airtightly installed at the connector portion. The connector portion includes: a base portion having a tubular shape; a tubular sleeve portion extending from the base portion and having a diameter smaller than a diameter of the base portion; and an airtightness release portion provided at an end portion of the sleeve portion and configured to release an airtight state between the connector portion and the water-resistant cap. The water-resistant cap includes: a tubular cap main body having a closed bottom at one side; and an abutting portion provided inside the cap main body and configured to abut on the sleeve portion. When the water-resistant cap is installed at the connector portion, the endoscope system takes any state of a first state where the abutting portion abuts on the sleeve portion on a side of the base portion with respect to the airtightness release portion to hold an airtightness between the water-resistant cap and the connector portion, and a second state where the abutting portion abuts on or faces the sleeve portion including the airtightness release portion to make non-airtight between the water-resistant cap and the connector portion.

In the endoscope system according to the present invention, the sleeve portion includes: an airtightness portion that extends from the base portion, has a tubular shape having a diameter smaller than the diameter of the base portion, and is configured to be airtightly coupled to the abutting portion; and a reduced diameter portion that has a tubular shape that extends from an end portion of the airtightness portion, the end portion of the airtightness portion being at a side different from the side of the base portion, the reduced diameter portion having an outer peripheral surface with a diameter decreasing toward a distal end, and the airtightness release portion is provided at the reduced diameter portion.

In the endoscope system according to the present invention, the airtightness release portion has a cutout shape where the sleeve portion is cut out from a distal end toward a proximal end.

In the endoscope system according to the present invention, the airtightness release portion has a chamfered end portion continuous with the distal end of the sleeve portion.

In the endoscope system according to the present invention, the airtightness release portion has a hole.

In the endoscope system according to the present invention, the airtightness release portion has a concave shape where a part of the outer peripheral surface is depressed to an inner peripheral side.

In the endoscope system according to the present invention, the airtightness release portion is formed such that a part of the outer peripheral surface is circumferentially depressed to the inner peripheral side.

### Advantageous Effects of Invention

The present invention provides an advantageous effect that a water-resistant cap can be easily removed from an electric connector portion of an endoscope whose inside is in a negative pressure state.

### Brief Description of Drawings

FIG. 1 is a drawing schematically illustrating an endoscope system according to a first embodiment of the present invention.
FIG. 2 is a perspective view schematically illustrating a first connector portion in the endoscope system according to the first embodiment of the present invention.
FIG. 3 is a perspective view illustrating an internal structure of a water-resistant cap in the endoscope system according to the first embodiment of the present invention.
FIG. 4 is a cross-sectional view illustrating a state where the water-resistant cap illustrated in FIG. 3 is installed at the first connector portion illustrated in FIG. 2.
FIG. 5A is a drawing that describes the installation of the water-resistant cap illustrated in FIG. 3 with respect to the first connector portion illustrated in FIG. 2.
FIG. 5B is a drawing that describes the installation of the water-resistant cap illustrated in FIG. 3 with respect to the first connector portion illustrated in FIG. 2.
FIG. 5C is a drawing that describes the installation of the water-resistant cap illustrated in FIG. 3 with respect to the first connector portion illustrated in FIG. 2.
FIG. 6 is a drawing that describes the installation of the water-resistant cap illustrated in FIG. 3 with respect to the first connector portion illustrated in FIG. 2.
FIG. 7 is a drawing that describes the installation of the water-resistant cap illustrated in FIG. 3 with respect to the first connector portion illustrated in FIG. 2.
FIG. 8 is a drawing that describes an installation of a conventional water-resistant cap with respect to a conventional electric connector portion.
FIG. 9 is a drawing schematically illustrating a first connector portion of an endoscope system according to a first modification of the first embodiment of the present invention.
FIG. 10 is a drawing schematically illustrating a first connector portion of an endoscope system according to a second modification of the first embodiment of the present invention.
FIG. 11 is a drawing schematically illustrating a first connector portion of an endoscope system according to a third modification of the first embodiment of the present invention.
FIG. 12 is a drawing schematically illustrating a first connector portion of an endoscope system according to a fourth modification of the first embodiment of the present invention.
FIG. 13 is a drawing schematically illustrating a first connector portion of an endoscope system according to a fifth modification of the first embodiment of the present invention.
FIG. 14 is a cross-sectional view schematically illustrating a part of the first connector portion in the endoscope system according to the fifth modification of the first embodiment of the present invention.
FIG. 15 is a drawing schematically illustrating a first connector portion of an endoscope system according to a sixth modification of the first embodiment of the present invention.
FIG. 16 is a drawing schematically illustrating a first connector portion of an endoscope system according to a seventh modification of the first embodiment of the present invention.
FIG. 17 is a drawing schematically illustrating a first connector portion of an endoscope system according to an eighth modification of the first embodiment of the present invention.
FIG. 18 is a drawing schematically illustrating an endoscope system according to a second embodiment of the present invention.
FIG. 19 is a drawing schematically illustrating a configuration of a main part of an operating unit in the endoscope system according to the second embodiment of the present invention.
FIG. 20 is a drawing schematically illustrating a configuration of a main part of the operating unit in the endoscope system according to the second embodiment of the present invention.
FIG. 21 is a drawing schematically illustrating a configuration of a main part of an inserted portion in the endoscope system according to the second embodiment of the present invention.
FIG. 22 is a drawing schematically illustrating a configuration of a main part of the inserted portion in the endoscope system according to the second embodiment of the present invention.
FIG. 23 is a drawing schematically illustrating a configuration of a main part of an ultrasound transducer in the endoscope system according to the second embodiment of the present invention.
FIG. 24 is a drawing schematically illustrating a configuration of a main part of a connector portion in the endoscope system according to the second embodiment of the present invention.
FIG. 25 is a cross-sectional view of the first connector portion taken along the line B-B illustrated in FIG. 24.
FIG. 26 is a drawing schematically illustrating a configuration of a main part of the operating unit in the endoscope system according to the second embodiment of the present invention.
FIG. 27 is a drawing schematically illustrating a configuration of a main part of the operating unit in the endoscope system according to the second embodiment of the present invention.
FIG. 28 is a drawing schematically illustrating a configuration of a main part of the operating unit in the endoscope system according to the second embodiment of the present invention.
FIG. 29 is a drawing schematically illustrating a configuration of a main part of the ultrasound transducer in the endoscope system according to the second embodiment of the present invention.
FIG. 30 is a partial cross-sectional view taken along the line C-C illustrated in FIG. 29.
FIG. 31 is a drawing schematically illustrating a configuration of a main part of the ultrasound transducer in the endoscope system according to the second embodiment of the present invention.
FIG. 32 is a drawing schematically illustrating a configuration of a main part of the ultrasound transducer in the endoscope system according to the second embodiment of the present invention.
FIG. 33 is a drawing schematically illustrating a configuration of a main part of the inserted portion in the endoscope system according to the second embodiment of the present invention.
FIG. 34 is a cross-sectional view of a cable sheath taken along the line D-D illustrated in FIG. 33. Description of Embodiments

The following describes modes for carrying out the invention (hereinafter referred to as "embodiments") with reference to the attached drawings.

### (First Embodiment)

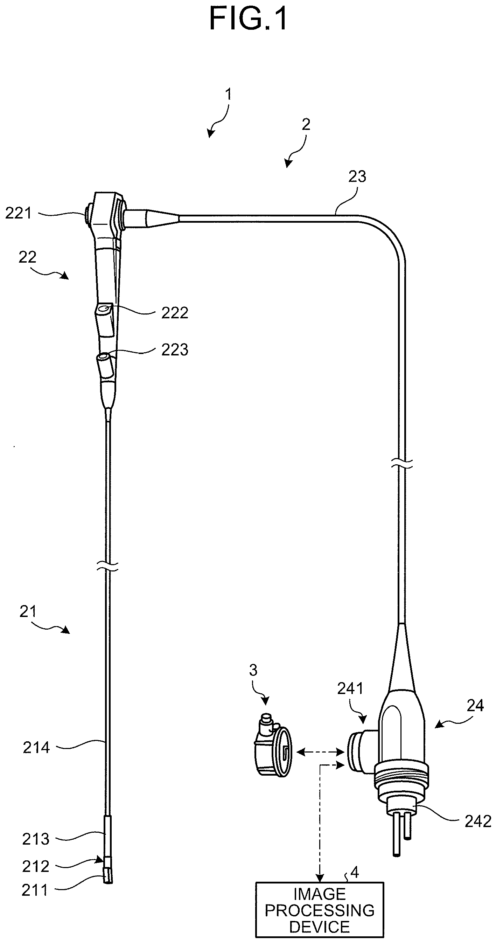

FIG. 1 is a drawing schematically illustrating an endoscope system 1 according to a first embodiment of the present invention. The endoscope system 1 is a system that uses a property of an ultrasonic sound wave to apply the ultrasonic sound wave to a subject, and then visualizes its reflection. This endoscope system 1, as illustrated in FIG. 1, includes an endoscope 2 and a water-resistant cap 3 to be installed at the endoscope 2. The endoscope 2 is an ultrasound endoscope, which is partially insertable into the subject and has a function that transmits the ultrasonic sound wave inside the subject and receives the reflection (an echo) reflected at the subject to output an echo signal. The endoscope 2 is configured to be coupled to an image processing device 4 that receives the echo signal to generate an ultrasound image corresponding to this echo signal, via a cable.

The endoscope 2 has a distal end portion including an ultrasound transducer 211 that converts an electrical pulse signal received from a processing device into an ultrasonic pulse to irradiate the subject with it and converts the ultrasonic echo reflected at the subject into an electrical echo signal expressed in a voltage change to output it. The ultrasound transducer 211 may be any of a convex transducer, a linear transducer, and a radial transducer. The endoscope 2 may be one that causes the ultrasound transducer to mechanically scan, or may be one that includes a plurality of elements in an array shape as the ultrasound transducer to cause them to mechanically scan by electronically switching elements related to transmitting and receiving or by delaying transmitting and receiving of the respective elements.

The endoscope 2, which usually includes an imaging optical system and an imaging element, is inserted into an alimentary canals (an esophagus, a stomach, a duodenum, and a large intestine) or respiratory organs (a trachea and a bronchus) of the subject to ensure capturing the alimentary canals, the respiratory organs, and their peripheral organs (a pancreas, a gallbladder, a bile duct, a biliary tract, a lymph node, a mediastinum organ, a blood vessel, and the like). It is not limited to the endoscope 2, and an ultrasonic probe that does not include the imaging optical system and the imaging element may be employed.

The endoscope 2, as illustrated in FIG. 1, includes an inserted portion 21, an operating unit 22, a universal cord 23, and a connector 24. A "distal end side" described below means a distal end side of the inserted portion 21. A "proximal end side" described below means a side separating from the distal end of the inserted portion 21.

The inserted portion 21 is a part inserted into the subject. This inserted portion 21, as illustrated in FIG. 1, includes a rigid member 212 that holds the ultrasound transducer 211 at a distal end, a curving portion 213 coupled to the proximal end side of the rigid member 212 to be configured to curve, and a flexible tube portion 214 coupled to the proximal end side of the curving portion 213 to have flexibility.

Here, inside the inserted portion 21, although a specific illustration is omitted, a light guide that transmits illuminating light supplied from a light source device (not illustrated) and a plurality of signal cables that transmit various signals are drawn, and a processing tool insertion path for inserting a treatment tool is formed.

The operating unit 22 is a part coupled to the proximal end side of the inserted portion 21 to accept various operations from a doctor or the like. The operations, for example, include an angle operation, an air supply and water supply operation, and a suction operation. This operating unit 22, as illustrated in FIG. 1, includes a curving knob 221 for curving operation on the curving portion 213. At the operating unit 22, a treatment tool insertion port 222 that communicates with the treatment tool insertion path formed inside the inserted portion 21 to insert the treatment tool into this treatment tool insertion path, and an air supply and water supply port 223 that communicates with an air supply and water supply path formed inside the inserted portion 21 to pass gas or liquid through this air supply and water supply path are formed.

Inside the universal cord 23, a plurality of signal cables that extend from the operating unit 22 to transmit various signals and a cable at which an optical fiber and the like that transmit the illuminating light supplied from the light source device are arranged are drawn.

The connector 24 is provided at a distal end of the universal cord 23. Then, the connector 24 includes a first connector portion 241 to which a connector portion of a scope cable that holds a plurality of signal cables is coupled, to be coupled to the above-described image processing device 4, and a second connector portion 242 to which a cable at which an optical fiber and the like are arranged is coupled, to be coupled to the light source device.

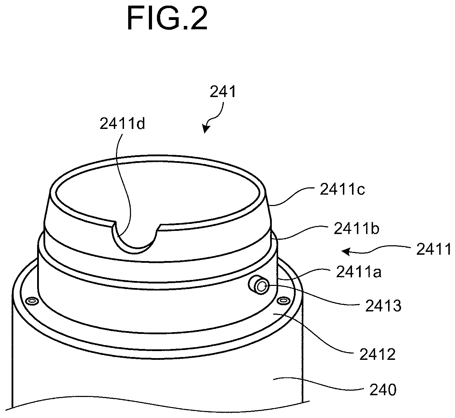

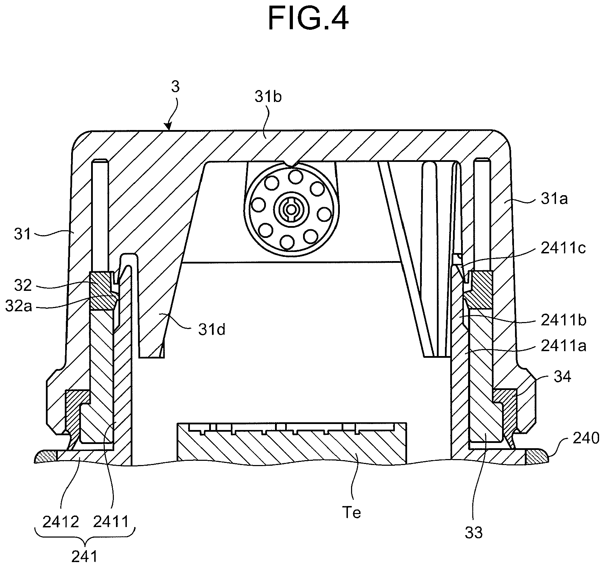

The following describes a structure of the first connector portion 241. FIG. 2 is a perspective view schematically illustrating the first connector portion 241 in the endoscope system 1 according to the first embodiment of the present invention. The first connector portion 241 is an electric connector for being electrically coupled to the plurality of signal cables internally mounted on the universal cord 23. The first connector portion 241, as illustrated in FIG. 2, has a cylindrical shape that covers a terminal Te (see FIG. 4) electrically coupled to the plurality of signal cables. Although a specific illustration is omitted, the terminal Te is arranged so as to airtightly obstruct an inside of the first connector portion 241 by an O-ring or the like.

the first connector portion 241 includes a coupling portion 2411 coupled to a connector portion (not illustrated) of the scope cable coupled to the image processing device 4, and a protruding portion 2412 formed by projecting outside from a proximal end (an end portion at a lower side in FIG. 2) of the coupling portion 2411. In the first connector portion 241, the protruding portion 2412 fits into an aperture formed at a connector casing 240 that constitutes the connector 24, and this aperture is airtightly obstructed by an O-ring or the like.

The coupling portion 2411, as illustrated in FIG. 2, includes a cylindrically-shaped base portion 2411a that extends from the protruding portion 2412, an airtightness portion 2411b that extends from the base portion 2411a toward a side different from a side of the protruding portion 2412, has a cylindrical shape having a diameter smaller than a diameter of the base portion 2411a, and is configured to be airtightly coupled to the water-resistant cap 3 described later, and a reduced diameter portion 2411c that has a cylindrical shape that extends from an end portion of the airtightness portion 2411b being at a side different from a side of the base portion 2411a and whose diameter that forms an outer peripheral surface decreases toward an distal end. At the reduced diameter portion 2411c, a cutout portion 2411d cut out from the distal end toward a proximal end is formed. The cutout portion 2411d forms an arc-shaped cutout surface that forms an edge end of the reduced diameter portion 2411c. In the first embodiment, the airtightness portion 2411b and the reduced diameter portion 2411c constitute a sleeve portion.

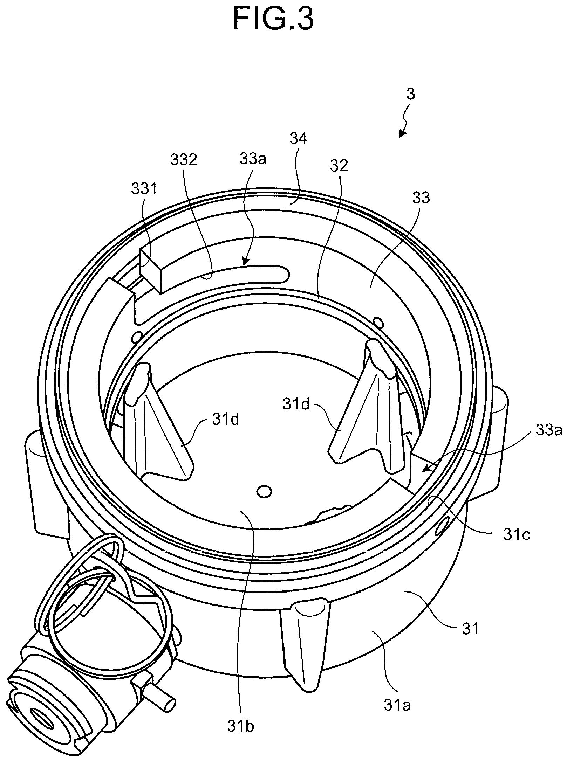

At the base portion 2411a, two locking pins 2413 for locking the water-resistant cap 3 (see FIG. 3) on the first connector portion 241 are installed so as to project from the outer peripheral surface.

On the endoscope 2, as described above, before and after the ultrasonic diagnostics inside the subject, the scope cable is removed from the first connector portion 241, and then, the sterilization treatment by an autoclave or the like and the disinfectant treatment by an antiseptic solution are performed. In this respect, In order to provide two functions: a watertight function that prevents liquid such as water vapor and the antiseptic solution from entering inside the first connector portion 241 and an airtight function that prevents sterilization gas from internally entering, the water-resistant cap 3 (see FIG. 3) is installed at the first connector portion 241.

FIG. 3 is a perspective view illustrating an internal structure of the water-resistant cap 3 installed at the first connector portion 241. FIG. 4 is a cross-sectional view illustrating a state where the water-resistant cap 3 has been installed at the first connector portion 241. The water-resistant cap 3, as illustrated in FIG. 3 and FIG. 4, includes a cap main body 31, a first packing 32 (an abutting portion), a cam main body 33, and a second packing 34.

The cap main body 31, as illustrated in FIG. 3 and FIG. 4, includes a cylindrically-shaped sidewall portion 31a that allows the coupling portion 2411 to be inserted and a bottom portion 31b that obstructs one end of the sidewall portion 31a, and is formed in a cylindrical shape with a closed bottom at one side having an aperture portion 31c at the other end of the sidewall portion 31a. On an inner surface of the bottom portion 31b (a bottom surface of the cap main body 31), ribs 31d are provided upright. Then, as illustrated in FIG. 4, when the water-resistant cap 3 is installed at the first connector portion 241, this rib 31d houses a part of the coupling portion 2411 in a hollow space formed by this rib 31d and the sidewall portion 31a.

The first packing 32, which has a ring shape, is installed on an inner surface of the sidewall portion 31a. As illustrated in FIG. 4, the first packing 32 includes a convex portion 32a that abuts on an outer peripheral surface of the coupling portion 2411, specifically, an outer peripheral surface of the airtightness portion 2411b to keep airtightness of the inside of the first connector portion 241 when the water-resistant cap 3 is installed at the first connector portion 241.

The cam main body 33, which has a cylindrical shape that allows the coupling portion 2411 to be inserted, is installed at a side of the aperture portion 31c on the inner surface of the sidewall portion 31a. At this cam main body 33, as illustrated in FIG. 3, two locking grooves 33a into which the two locking pins 2413 are each inserted are formed.

Each of the two locking grooves 33a, as illustrated in FIG. 3, has an approximately L shape including a first locking groove 331 that extends from one end (an end portion at the aperture portion 31c side) toward the other end (an end portion at a side of the bottom portion 31b), and a second locking groove 332 that extends from the first locking groove 331 in a rotation direction centering a center axis (not illustrated) of the cam main body 33 and with being inclined toward the bottom portion 31b side. That is, in the first embodiment, as a locking structure of the first connector portion 241 and the water-resistant cap 3, a bayonet-type locking structure is employed.

The second packing 34, which has a ring shape, is installed at the aperture portion 31c in a state where a part of the second packing 34 is inserted into a clearance between the cam main body 33 and the inner surface of the sidewall portion 31a. Then, as illustrated in FIG. 4, when the water-resistant cap 3 is installed at the first connector portion 241, the second packing 34 abuts on the protruding portion 2412 to keep the airtightness of the inside of the first connector portion 241.

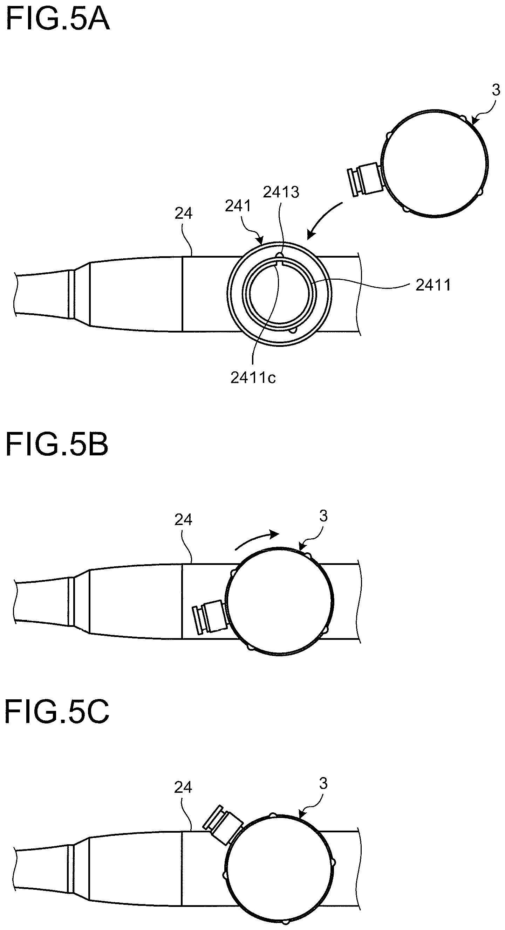

Subsequently, the installation of the water-resistant cap 3 with respect to the first connector portion 241 will be described with reference to FIGS. 5 to 8. FIGS. 5A to 5C are drawings that describe the installation of the water-resistant cap 3 with respect to the first connector portion 241.

When the water-resistant cap 3 is installed at the first connector portion 241, first, as illustrated in FIG. 5A, the water-resistant cap 3 is fitted in the first connector portion 241. In this respect, the locking pin 2413 becomes in a state housed in the first locking groove 331 (hereinafter, referred to as a first locking state).

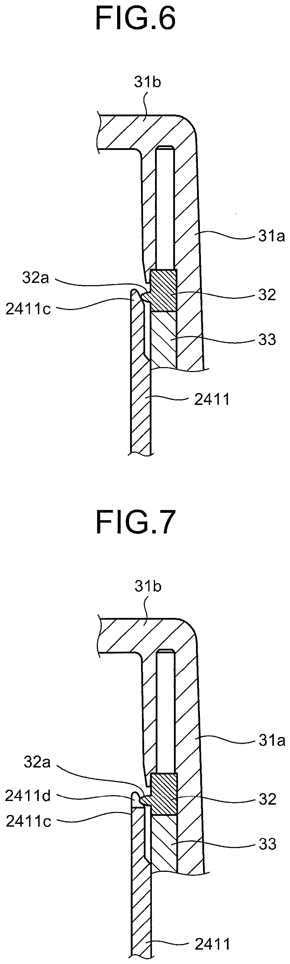

FIG. 6 is a drawing that describes the installation of the water-resistant cap 3 with respect to the first connector portion 241, and a cross-sectional view illustrating a positional relation between the first packing 32 and the coupling portion 2411 in the first locking state. In the first locking state, as illustrated in FIG. 6, the convex portion 32a of the first packing 32 abuts on the reduced diameter portion 2411c, or is arranged at a position faced the reduced diameter portion 2411c. In view of this, the first locking state makes non-airtight between the first connector portion 241 and the water-resistant cap 3, and does not become in the airtight state.

Thereafter, as illustrated in FIG. 5B, the water-resistant cap 3 is rotated along a forming direction of the second locking groove 332 with respect to the first connector portion 241. In this respect, the locking pin 2413 moves along the second locking groove 331. Accordingly, the water-resistant cap 3 is pushed into a direction approaching the first connector portion 241. Hereinafter, a locking state where the locking pin 2413 has moved to an end portion of the second locking groove 332 being an end portion at a side different from a side of the first locking groove 331 is referred to as a second locking state (see FIG. 5C).

In the second locking state, as illustrated in FIG. 4, the convex portion 32a of the first packing 32 is arranged at a position abutting on the outer peripheral surface of the airtightness portion 2411b to be in a state where the airtightness inside the first connector portion 241 is kept. In this second locking state, a negative pressure state is made, for example, by a suction inside of the endoscope 2, to sterilize the endoscope 2.

After the sterilization treatment is terminated, an operator rotates the water-resistant cap 3 to change the state from the second locking state to the first locking state, and then removes the water-resistant cap 3 from the first connector portion 241.

FIG. 7 is a drawing that describes the installation of the water-resistant cap 3 with respect to the first connector portion 241, and a cross-sectional view illustrating a positional relation between the first packing 32 and the coupling portion 2411 in the first locking state after the sterilization treatment. In the first locking state after the sterilization treatment or the disinfectant treatment, as illustrated in FIG. 7, a part of the convex portion 32a of the first packing 32 is arranged at a position faced the cutout portion 2411d of the reduced diameter portion 2411c. Thus, the airtight state between the convex portion 32a and the coupling portion 2411 is released to return the negative pressure state of the first connector portion 241 to a normal pressure state. Thus, the cutout portion 2411d functions as an airtightness release portion configured to release the airtight state between the first connector portion 241 and the water-resistant cap 3.

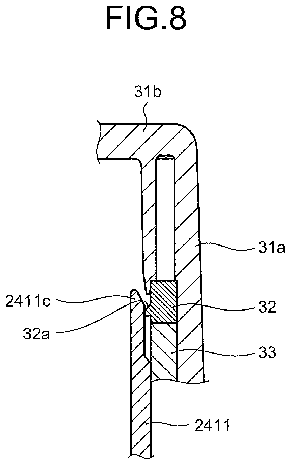

FIG. 8 is a drawing that describes an installation of a conventional water-resistant cap with respect to a conventional electric connector portion. Compared to FIG. 7, in a case of the reduced diameter portion 2411c that does not include the cutout portion 2411d, when the water-resistant cap 3 is rotated to change the state from the second locking state to the first locking state, the convex portion 32a is pulled to a side of the reduced diameter portion 2411c in a state where the convex portion 32a is attached to the airtightness portion 2411b. Thus, for example, as illustrated in FIG. 8, the convex portion 32a is attached to the airtightness portion 2411b or the reduced diameter portion 2411c to be in a state where the airtightness is kept. In view of this, even when the water-resistant cap 3 is rotated to make the first locking state, the airtight state (the negative pressure state) of the first connector portion 241 remains to be kept. Thus, sometimes, the water-resistant cap 3 cannot be removed from the first connector portion 241.

According to the above-described first embodiment of the present invention, in the connector portion (the first connector portion 241) of the endoscope 2, the cutout portion 2411d where a part of the reduced diameter portion 2411c is cut out is formed. When the water-resistant cap 3 is rotated to change the state from the second locking state to the first locking state, the airtight state between the convex portion 32a of the first packing 32 and the reduced diameter portion 2411c is released, thus ensuring easily removing a water-resistant cap from an endoscope whose inside is in the negative pressure state.

According to the first embodiment, the cutout portion 2411d is formed at the reduced diameter portion 2411c, thus, as described above, ensuring easily removing the water-resistant cap from an electric connector portion of the endoscope, and ensuring surely keeping the airtight state between the water-resistant cap 3 and the first connector portion 241 when the water-resistant cap 3 becomes in the second locking state, specifically the airtight state between the convex portion 32a and the airtightness portion 2411b.

According to the first embodiment, the diameter of the reduced diameter portion 2411c is reduced toward the distal end. Thus, the reduced diameter portion 2411c functions as a guide unit when the convex portion 32a and the cam main body 33 are installed at the first connector portion 241, and damage of the convex portion 32a in accordance with this installation can be restrained.

In the above-described first embodiment, the cutout portion 2411d is described as one where the reduced diameter portion 2411c is cut out in an arc shape (an approximately semicircular shape). However, the cutout portion 2411d is not limited to this. The following describes modifications of the first embodiment of the present invention.

### (First Modification of First Embodiment)

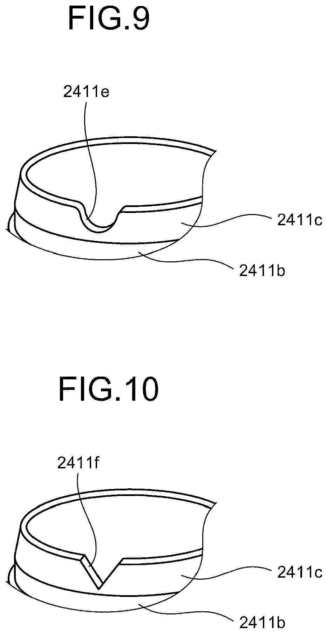

Subsequently, a first modification of the first embodiment of the present invention will be described. FIG. 9 is a drawing schematically illustrating the first connector portion 241 in the endoscope system 1 according to the first modification of the first embodiment of the present invention. The first modification has a shape where an edge end of the cutout portion 2411d is chamfered. A cutout portion 2411e according to the first modification, as illustrated in FIG. 9, has a shape where the reduced diameter portion 2411c is cut out from the distal end toward the proximal end, and furthermore, both ends of the cutout portion 2411e continuous with the distal end of the reduced diameter portion 2411c in a circumferential direction are chamfered. According to the first modification, when the water-resistant cap 3 is installed at the first connector portion 241, the damage of the convex portion 32a in accordance with this installation can be further surely restrained.

### (Second Modification of First Embodiment)

Subsequently, a second modification of the first embodiment of the present invention will be described. FIG. 10 is a drawing schematically illustrating the first connector portion 241 in the endoscope system 1 according to the second modification of the first embodiment of the present invention. A cutout portion 2411f according to the second modification, as illustrated in FIG. 10, has a shape where the reduced diameter portion 2411c is cut out from the distal end toward the proximal end in a V shape.

### (Third Modification of First Embodiment)

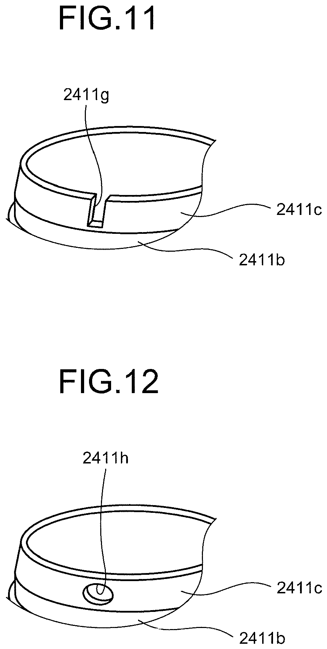

Subsequently, a third modification of the first embodiment of the present invention will be described. FIG. 11 is a drawing schematically illustrating the first connector portion 241 in the endoscope system 1 according to the third modification of the first embodiment of the present invention. A cutout portion 2411g according to the third modification, as illustrated in FIG. 11, has a shape where the reduced diameter portion 2411c is cut out from the distal end toward the proximal end in an approximately U shape.

### (Fourth Modification of First Embodiment)

Subsequently, a fourth modification of the first embodiment of the present invention will be described. FIG. 12 is a drawing schematically illustrating the first connector portion 241 in the endoscope system 1 according to the fourth modification of the first embodiment of the present invention. A cutout portion 2411h according to the fourth modification, as illustrated in FIG. 12, has a hole shape that communicates between an inner surface and the outer peripheral surface of the reduced diameter portion 2411c.

### (Fifth Modification of First Embodiment)

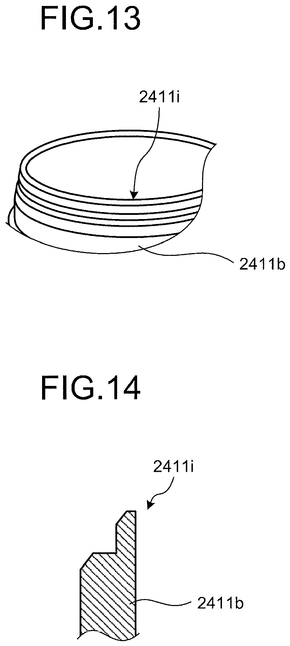

Subsequently, a fifth modification of the first embodiment of the present invention will be described. FIG. 13 is a drawing schematically illustrating the first connector portion 241 in the endoscope system 1 according to the fifth modification of the first embodiment of the present invention. FIG. 14 is a cross-sectional view schematically illustrating a part of the first connector portion 241 in the endoscope system 1 according to the fifth modification of the first embodiment of the present invention. The cross-sectional view illustrated in FIG. 14 is a cross-sectional view whose cut surface is a planar surface parallel to a center axis (a cylindrical axis) of the coupling portion 2411. A cutout portion 2411i according to the fifth modification, as illustrated in FIG. 13, has a concave shape where a part of the above-described outer peripheral surface of the reduced diameter portion 2411c is depressed to an inner peripheral side over the circumferential direction. In other words, the cutout portion 2411i has a shape where an outer surface of the reduced diameter portion 2411c (see FIG. 2) is cut out over the circumferential direction. The cutout portion 2411i, as illustrated in FIG. 14, has a stepped shape in a sectional side view viewed from a direction perpendicular to the center axis of the coupling portion 2411. According to the fifth modification, a region where this reduced diameter portion 2411c and the convex portion 32a are contactless in the first locking state is large compared with the above-described first embodiment, thus ensuring further surely returning the state to the normal pressure state. In the fifth modification, it has been described that the surface at the depressed part of the cutout portion 2411i has a shape orthogonally bent (see FIG. 14). However, this may have a shape that forms a curved surface.

### (Sixth Modification of First Embodiment)

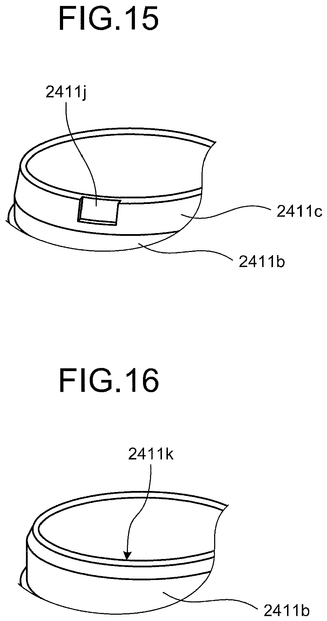

Subsequently, a sixth modification of the first embodiment of the present invention will be described. FIG. 15 is a drawing schematically illustrating the first connector portion 241 in the endoscope system 1 according to the sixth modification of the first embodiment of the present invention. A cutout portion 2411j according to the sixth modification, as illustrated in FIG. 15, has a concave shape where a part of the outer peripheral surface of the reduced diameter portion 2411c is depressed to the inner peripheral side.

### (Seventh Modification of First Embodiment)

Subsequently, a seventh modification of the first embodiment of the present invention will be described. FIG. 16 is a drawing schematically illustrating the first connector portion 241 in the endoscope system 1 according to the seventh modification of the first embodiment of the present invention. A reduced diameter portion 2411k according to the seventh modification, as illustrated in FIG. 16, is formed such that, compared with the above-described reduced diameter portion 2411c, a length in an extending direction is shortened, and an inclined angle between the outer peripheral surface of the airtightness portion 2411b and the outer peripheral surface of this reduced diameter portion 2411k is increased. In this reduced diameter portion 2411k, the outer peripheral surface, which is an inclined surface, functions as an airtightness release portion that releases the airtight state between the first connector portion 241 and the water-resistant cap 3.

### (Eighth Modification of First Embodiment)

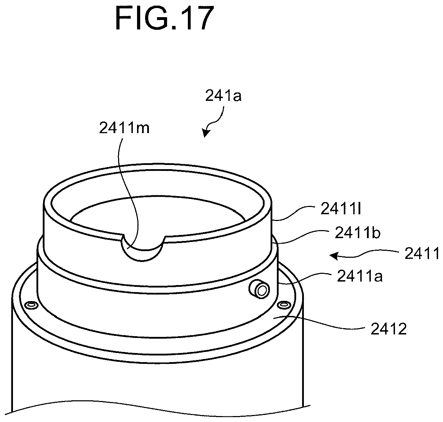

Subsequently, an eighth modification of the first embodiment of the present invention will be described. FIG. 17 is a drawing schematically illustrating a first connector portion 241a in the endoscope system 1 according to the eighth modification of the first embodiment of the present invention. The first connector portion 241a according to the eighth modification includes an expanded diameter portion 24111, instead of the above-described reduced diameter portion 2411c. The expanded diameter portion 24111 has a cylindrical shape that extends from the end portion of the airtightness portion 2411b, the end portion at the side different from the base portion 2411a side, and has a diameter that forms an inner surface increases toward the distal end. A cutout portion 2411m, similar to the above-described cutout portion 2411d, has a cutout shape where the expanded diameter portion 24111 is cut out from a distal end toward a proximal end. The cutout portions or the reduced diameter portions according to the above-described first to seventh modifications may be applied to this. The eighth modification, for example, can obtain the above-described advantageous effect when the first packing 32 is formed at a side of the rib 31d.

In the above-described first embodiment and first to fourth, sixth, and eighth modifications, it has been described that one cutout portion is formed. However, a plurality of cutout portions may be provided. When the plurality of cutout portions are provided, the respective cutout portions may have an identical size or different sizes, and, for example, may be a combination of those in the first embodiment and the first to seventh modifications as necessary.

In the above-described first embodiment and first to eighth modifications, it has been described that the endoscope 2 is an ultrasound endoscope where a part of it can be inserted into the subject, and having the function that transmits the ultrasonic pulse toward the subject and receives the ultrasonic echo reflected at the subject to output the echo signal and the function that captures the inside of the subject to output the image signal. However, for example, the above-described structures of the electric connector portion and the water-resistant cap may be applied to an endoscope that has only the function that captures the inside of the subject to output the image signal and an endoscope that has only the function that transmits the ultrasonic pulse toward the subject and receives the ultrasonic echo reflected at the subject to output the echo signal.

### (Second Embodiment)

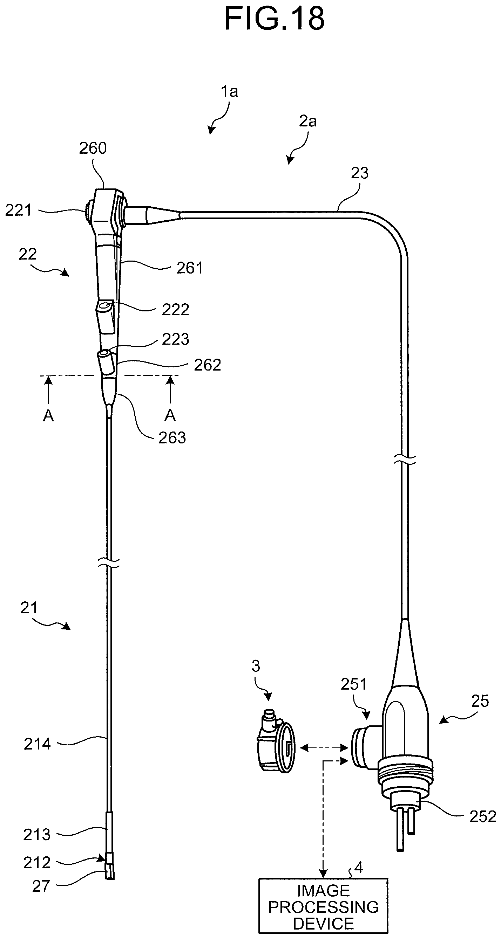

Subsequently, a second embodiment of the present invention will be described. FIG. 18 is a drawing schematically illustrating an endoscope system according to the second embodiment of the present invention. An endoscope system 1a is a system that performs the ultrasonic diagnostics inside the subject such as a human using an ultrasound endoscope. This endoscope system 1a, as illustrated in FIG. 18, includes an endoscope 2a, the water-resistant cap 3 installed at the endoscope 2a, and the image processing device 4.

The endoscope 2a, as illustrated in FIG. 18, includes the inserted portion 21, the operating unit 22, the universal cord 23, and a connector 25.

In the second embodiment, the operating unit 22, as illustrated in FIG. 18, includes the curving knob 221, and includes a first operating unit 260 removably coupled to the universal cord 23, a second operating unit 261 at which the treatment tool insertion port 222 is provided, a third operating unit 262 at which the air supply and water supply port 223 is provided, and a fourth operating unit 263 coupled to the third operating unit 262 at one end and coupled to the inserted portion 21 at the other end.

The connector 25 is provided at a distal end of the universal cord 23. Then, the connector 25 includes a first connector portion 251 to which a connector portion of a scope cable that holds a plurality of signal cables is coupled, to be configured to be coupled to the above-described image processing device 4 and water-resistant cap 3, and a second connector portion 252 to which a cable at which an optical fiber and the like are arranged is coupled, to be coupled to the light source device.

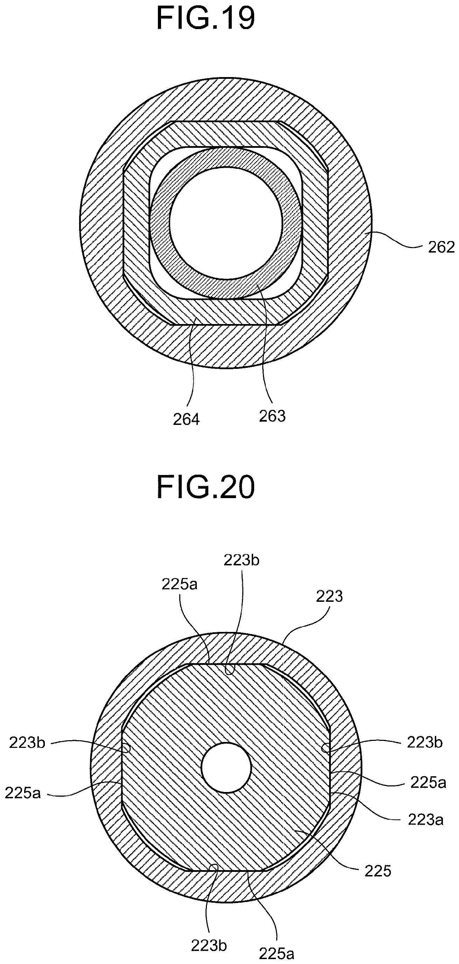

FIG. 19 is a drawing schematically illustrating a configuration of a main part of the operating unit in the endoscope system according to the second embodiment of the present invention, and a partial cross-sectional view taken along the line A-A illustrated in FIG. 18. As illustrated in FIG. 19, the third operating unit 262 has an end portion at a side coupled to the fourth operating unit 263, and at this end portion, a rotation prevention member 264 that prevents the fourth operating unit 263 from rotating around a center axis (a longitudinal axis of the operating unit 22) of the third operating unit 262 is provided. Here, the third operating unit 262 forms a hole having an approximately octagon shape. The fourth operating unit 263 has outer peripheries at parts coupled to the third operating unit 262, and at theses outer peripheries, planar portions are formed. The rotation prevention member 264 has an approximately-octagon-shaped ring shape, thus ensuring preventing the fourth operating unit 263 from rotating around the center axis of the third operating unit 262. It is not limited to the above-described octagon shape, and it is possible to employ a shape having at least one planar portion and a shape that does not have continuous rotation symmetry, such as an elliptical shape.

FIG. 20 is a drawing schematically illustrating a configuration of a main part of the operating unit in the endoscope system according to the second embodiment of the present invention, and a cross-sectional view whose cut surface is a planar surface perpendicular to an extending direction of the air supply and water supply port 223. As illustrated in FIG. 20, the air supply and water supply port 223 holds a water filling base 225 for air supply and water supply, for example, sending liquid into a balloon provided at the distal end (the ultrasound transducer 211) of the inserted portion 21. Here, inside the air supply and water supply port 223, hole 223a having planar portions 223b is formed. The water filling base 225 has outer peripheries at parts coupled to the hole 223a, and these outer peripheries form shapes having planar portions 225a provided corresponding to the planar portions 223b of the hole 223a. When the air supply and water supply port 223 is coupled to the water filling base 225, they are coupled one another with the above-described planar portions 223b and 225a contacting one another, thus ensuring restraining the water filling base 225 from rotating around a center axis of the hole 223a. As described above, it is only necessary that the hole 223a and the water filling base 225 have a shape that does not have the continuous rotation symmetry.

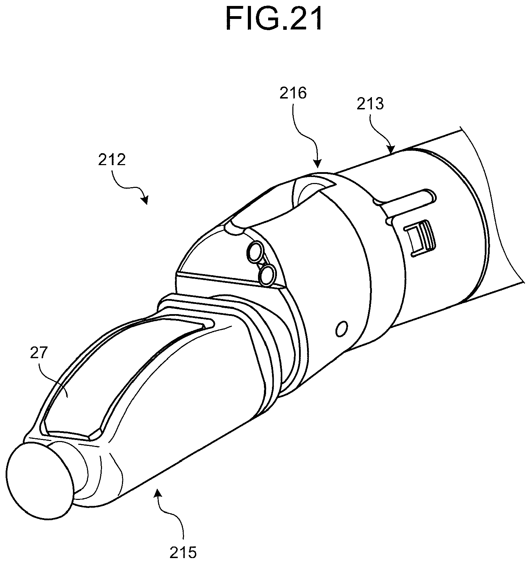

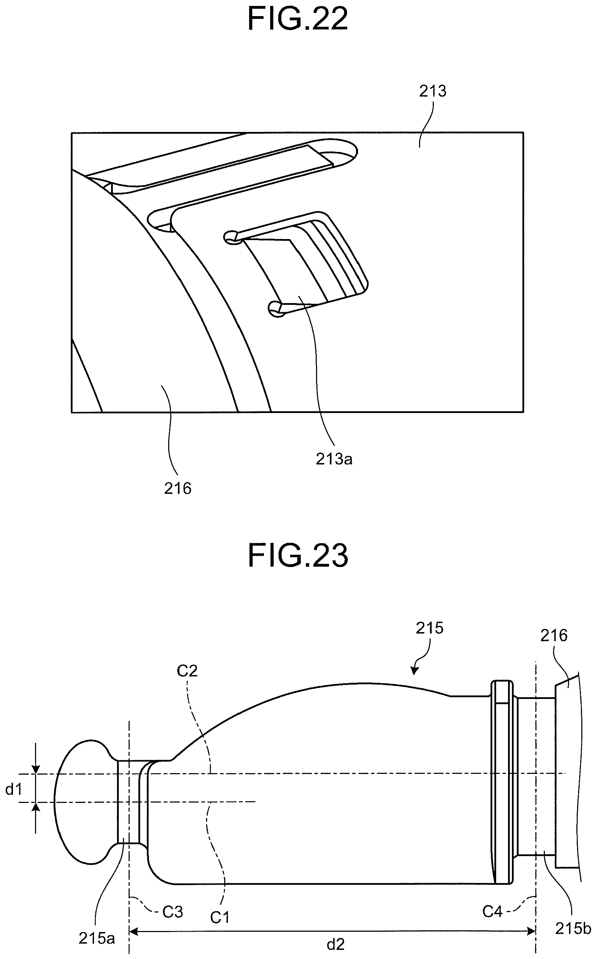

FIG. 21 is a drawing schematically illustrating a configuration of a main part of the inserted portion in the endoscope system according to the second embodiment of the present invention, and a perspective view illustrating a part of the rigid member 212 and the curving portion 213. FIG. 22 is a drawing schematically illustrating a configuration of a main part of the inserted portion in the endoscope system according to the second embodiment of the present invention, and a drawing illustrating an enlarged coupling part of the rigid member 212 and the curving portion 213 among the configuration illustrated in FIG. 21. FIGS. 21 and 22 illustrate a configuration where an outer coat of the inserted portion 21 has been removed. The rigid member 212 includes an ultrasonic functional portion 215 that has an ultrasound transducer 27, a holding port continuous with the ultrasonic functional portion 215 at one end and coupled to the curving portion 213 at the other end to hold an objective lens and an illumination lens, and a tubular endoscope functional portion 216 at which a treatment tool injection hole coupled to the treatment tool insertion port 222 is formed. The endoscope functional portion 216 is housed in the curving portion 213 to fit to it, thus being coupled to the curving portion 213.

The curving portion 213 has a part coupled to the endoscope functional portion 216, and at this part, a caulking portion 213a is formed. The caulking portion 213a has a scale shape formed such that a C-shaped slit is formed at a part of the curving portion 213. This caulking portion 213a is pressed onto the endoscope functional portion 216, thus ensuring preventing the rigid member 212 from exiting from the curving portion 213.

FIG. 23 is a drawing schematically illustrating a configuration of a main part of the ultrasound transducer in the endoscope system according to the second embodiment of the present invention, and a drawing of the inserted portion 21 viewed from a direction perpendicular to a longitudinal axis of the inserted portion 21. As illustrated in FIG. 23, at the ultrasonic functional portion 215, a first balloon holding groove 215a that is provided at the distal end side of the ultrasound transducer 27 and has a groove shape formed along the circumferential direction, and a second balloon holding groove 215b that is provided at the proximal end side of the ultrasound transducer 27 and has a groove shape formed along the circumferential direction are formed.

In the second embodiment, an axis C1 passes through a center of the first balloon holding groove 215a and is parallel to a longitudinal axis of the ultrasonic functional portion 215. An axis C2 passes through a center of the second balloon holding groove 215b and is parallel to the longitudinal axis of the ultrasonic functional portion 215. A distance d1 is a distance between the axis C1 and the axis C2. An axis C3 passes through a center portion in the longitudinal axis direction of the ultrasonic functional portion 215, of the first balloon holding groove 215a and is perpendicular to this longitudinal axis. An axis C4 passes through a center portion in the longitudinal axis direction of the ultrasonic functional portion 215, of the second balloon holding groove 215b and is perpendicular to this longitudinal axis. A distance d2 is a distance between the axis C3 and the axis C4. In this case, the distances d1 and d2 satisfy a relation that d1/d2 < 0.1. The satisfaction of this relation, when one end and the other end of a balloon are arranged at the first balloon holding groove 215a and the second balloon holding groove 215b, restrains a one-sided bulge and the like to ensure efficiently expanding or contracting the balloon.

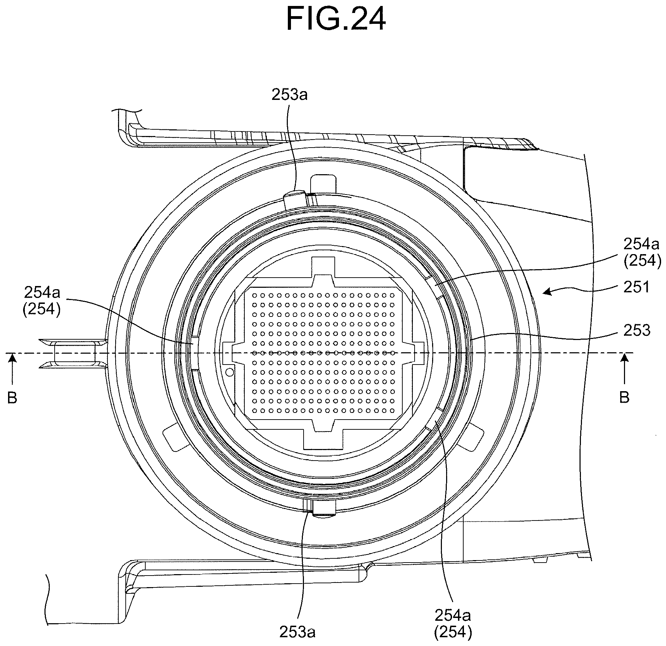

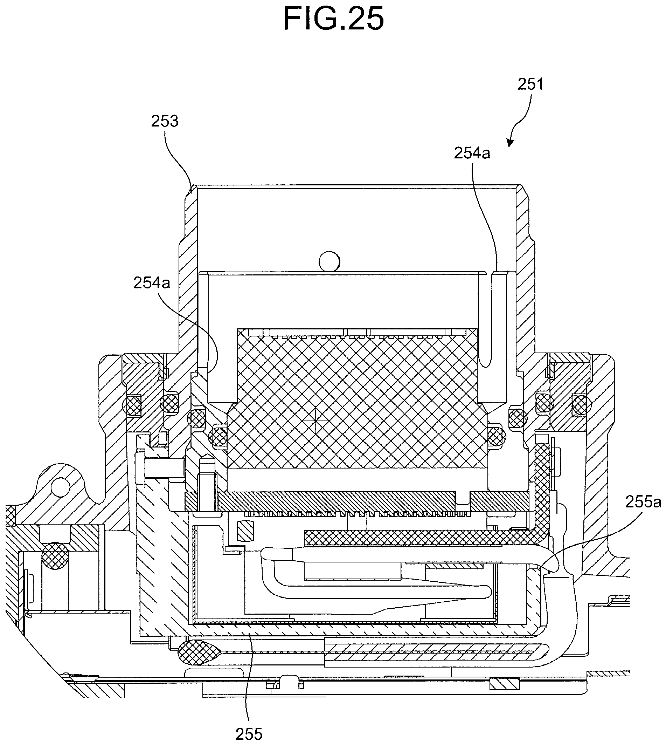

FIG. 24 is a drawing schematically illustrating a configuration of a main part of the connector portion in the endoscope system according to the second embodiment of the present invention, and a drawing illustrating a configuration of the first connector portion 251. FIG. 25 is a cross-sectional view of the first connector portion taken along the line B-B illustrated in FIG. 24. The first connector portion 251 includes an outer cylinder 253 having a tubular shape, and an inner cylinder 254 provided inside the outer cylinder and is formed such that a plurality of extending portions 254a that extend in a central axis direction are formed along the circumferential direction. At an outer periphery of the outer cylinder 253, one or a plurality of (two in the second embodiment) base pins 253a that project in a radial direction are provided. The first connector portion 251 is configured to be coupled to only the connector portion of the scope cable having patterns corresponding to coupling patterns by disposition of the base pins 253a and the extending portions 254a, or the water-resistant cap 3. This can prevent the first connector portion 251 from being improperly coupled to the connector portion of the scope cable and the water-resistant cap 3.

As illustrated in FIG. 25, at the first connector portion 251, an insulating member 255 that is formed using resin having an insulation property, has a tubular shape with a closed bottom at one-side, and includes a cutout portion 255a formed such that a part of a side surface of the insulating member 255 is cut out is provided. This insulating member 255 maintains ease of assembly of the first connector portion 251, and the cable is arranged via the cutout portion 255a where a part of the side surface that extends from a bottom portion, thus ensuring a creepage distance to have more surely the insulating property between a cable of a patient ground and a cable that transmits the ultrasonic signal.

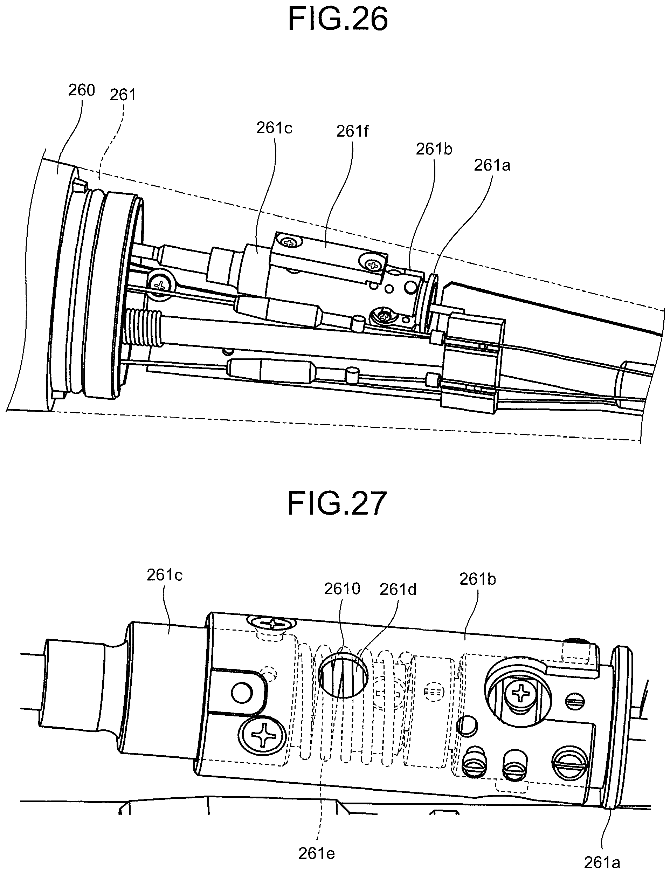

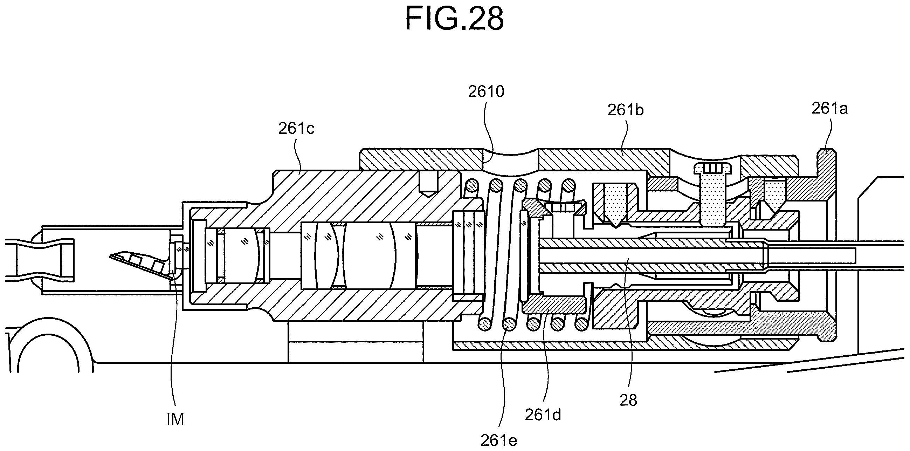

FIGS. 26 to 28 are drawings schematically illustrating a configuration of a main part of the operating unit in the endoscope system according to the second embodiment of the present invention, and a drawing where an outer coat of the second operating unit 261 has been removed. FIG. 28 is a cross-sectional view whose cut surface is a planar surface that passes through an image quality adjustment hole 2610 illustrated in FIG. 27 and is parallel to an optical axis direction of a lens. The second operating unit 261 internally includes a fiber holding portion 261a that extends from the distal end of the inserted portion 21 to hold an optical fiber 28 for optically transmitting the image signal, an optical element holding portion 261c that holds a plurality of lenses, and a plurality of spacer rings and imaging elements IM, a lens holding portion 261d supported to the fiber holding portion 261a to hold at least one lens, a coiled spring 261e that interposes between the fiber holding portion 261a and the optical element holding portion 261c, and a cover 261b that holds the fiber holding portion 261a and the optical element holding portion 261c.

At the cover 261b, the image quality adjustment hole 2610 is formed between the fiber holding portion 261a and the optical element holding portion 261c, and corresponding to an arranging position of the coiled spring 261e. The image quality adjustment hole 2610 is configured to be sealed by a sealing member 261f removably provided at the cover 261b. The sealing member 261f is fixedly secured to the cover 261b, for example, by screwing. The seal of the image quality adjustment hole 2610 by the sealing member 261f ensures preventing impurities such as waste and dust from attaching a lens surface, and light from an outside of the cover 261b from entering the plurality of lenses and imaging elements IM held by the optical element holding portion 261c.

Usually, when the endoscope 2a is used, as illustrated in FIG. 26, the endoscope 2a is used in a state sealed by the sealing member 261f. On the other hand, in order to adjust the image quality, when the light is entered into the plurality of lenses and imaging elements IM held by the optical element holding portion 261c, the outer coat of the second operating unit 261 is removed, and the sealing member 261f is removed, thus making a state that can take in the light from the image quality adjustment hole 2610 and between wire rods of the coiled spring 261e. This facilitates the image quality adjustment process.

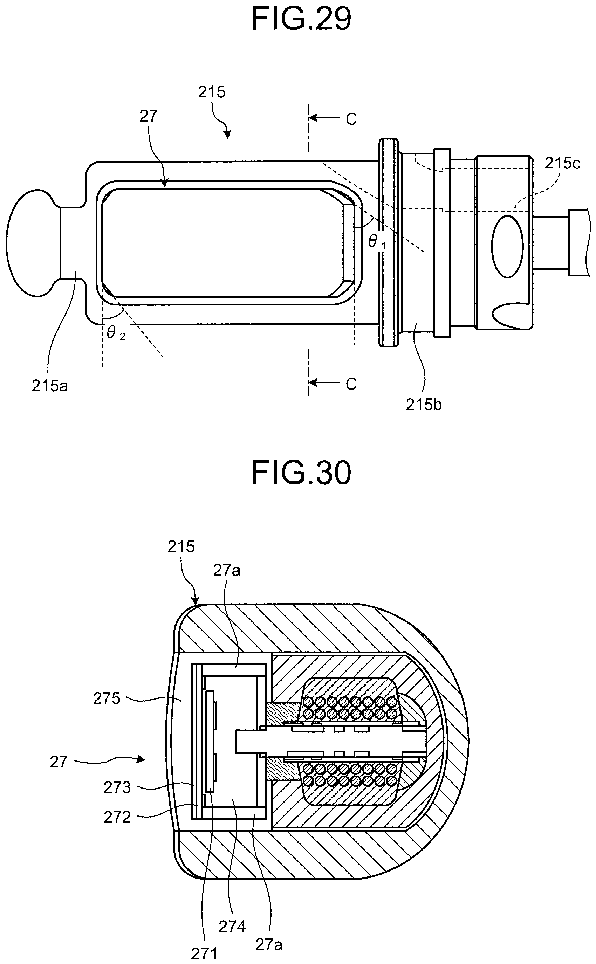

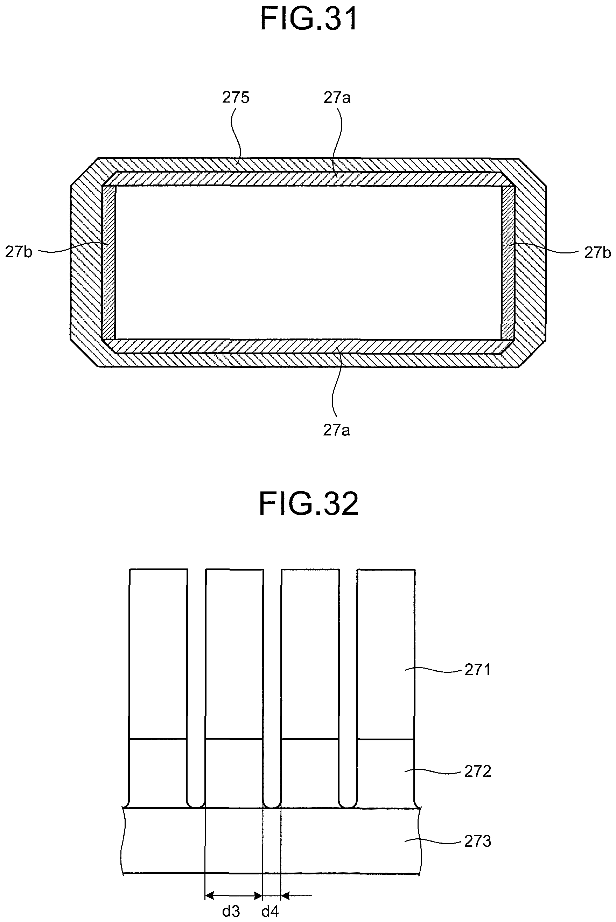

FIG. 29 is a drawing schematically illustrating a configuration of a main part of the ultrasound transducer in the endoscope system according to the second embodiment of the present invention, and a drawing illustrating a configuration of the ultrasonic functional portion 215 removed from the endoscope functional portion 216. FIG. 30 is a partial cross-sectional view taken along the line C-C illustrated in FIG. 29. FIG. 31 is a drawing schematically illustrating a configuration of a main part of the ultrasound transducer in the endoscope system according to the second embodiment of the present invention, and a cross-sectional view whose cut surface is a planar surface parallel to a paper surface of FIG. 29 and illustrating an acoustic lens and first and second wall portions. In the second embodiment, it is described that the ultrasound transducer 27 is a convex-type ultrasound transducer as illustrated in FIG. 21 and a one-dimensional array (a 1D array) where a plurality of piezoelectric elements 271 are arranged in line.

The ultrasound transducer 27 includes the plurality of piezoelectric elements 271 that have prismatic shapes and are formed by being arranged with longitudinal directions together, a plurality of first acoustic matching layers 272 each provided at an outer surface side of this ultrasound transducer 27 with respect to the piezoelectric elements 271, a second acoustic matching layer 273 provided at a side opposed to a side that contacts the piezoelectric elements 271, of the plurality of first acoustic matching layers 272, a backing material 274 provided at a side opposed to a side that contacts the first acoustic matching layers 272, of the piezoelectric elements 271 and filled between the piezoelectric elements 271, and an acoustic lens 275 provided at a side opposed to a side that contacts the first acoustic matching layers 272, of the second acoustic matching layer 273.

The first acoustic matching layers 272 and the second acoustic matching layer 273, which are formed using a super engineering plastic of epoxy resin, polyetherimide, and the like, match acoustic impedance between the piezoelectric elements 271 and an observation target so as to efficiently transmit sound (ultrasonic sound wave) to the piezoelectric elements 271 and the observation target. The formation of the first acoustic matching layers 272 and the second acoustic matching layer 273 using the super engineering plastic ensures improving chemical resistance and mechanical strength, compared with a case that uses the epoxy resin.

The backing material 274 attenuates unnecessary ultrasonic vibration generated by operation of the piezoelectric elements 271. The backing material 274 is formed using a material whose attenuation rate is large, for example, the epoxy resin where a filler such as alumina and zirconia is dispersed, and a rubber where the above-described filler is dispersed.

Here, the backing material 274 is formed such that liquid material for the backing material is cast into a hollow space formed by a first wall portion 27a and a second wall portion 27b (see FIG. 31) to be hardened. The first wall portion 27a and the second wall portion 27b are formed using polyphenylene ether. This ensures thinning thicknesses compared with a case that uses glass epoxy resin with a copper foil. The first wall portion 27a and the second wall portion 27b do not include metal, thus eliminating a need for wiring with a housing and a ground to facilitate the fabrication.

As illustrated in FIG. 29, at an outer edge shape of the ultrasound transducer 27, an angle θ₁ that forms a corner portion closest to a balloon pipe line 215c formed at the ultrasonic functional portion 215 is formed larger than angles (for example, an angle θ₂) of other corner portions. Thus, this can restrain thinning of a wall thickness of the housing of the ultrasonic functional portion 215 by the balloon pipe line 215c to ensure strength of the housing. This can also improve storing ability of the components inside the ultrasonic functional portion 215.

FIG. 32 is a drawing schematically illustrating a configuration of a main part of the ultrasound transducer in the endoscope system according to the second embodiment of the present invention, and a drawing illustrating an array of the piezoelectric elements 271, the first acoustic matching layers 272, and the second acoustic matching layer 273. When an element width of the piezoelectric element 271 in an arranging direction (a scanning direction) of the plurality of piezoelectric elements 271 is d3, and a groove width between the piezoelectric elements 271 adjacent to one another in the arranging direction is d4, a relation that d4/d3 < 0.4 is satisfied. The satisfaction of this relation maintains the number of elements and a pitch and reduces a curvature radius of the ultrasound transducer 27 to restrain deterioration of the image quality, thus ensuring downsizing the ultrasound transducer 27.

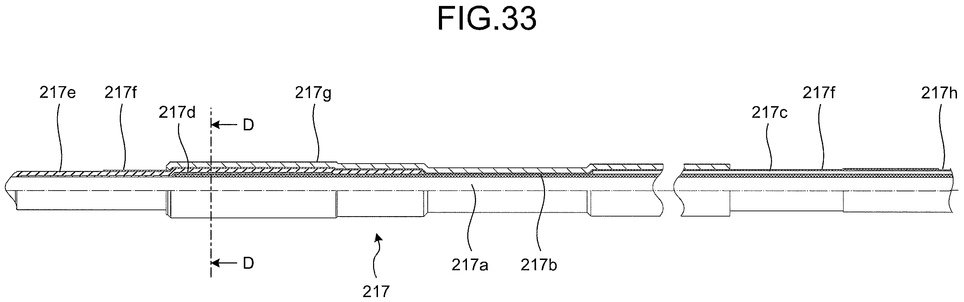

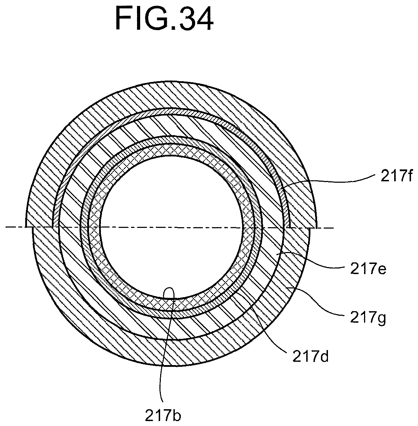

FIG. 33 is a drawing schematically illustrating a configuration of a main part of the inserted portion in the endoscope system according to the second embodiment of the present invention, and a partial cross-sectional view illustrating a configuration at the distal end side of a cable that contains one or a plurality of signal cables that extend from the ultrasound transducer 27, as one example. FIG. 34 is a cross-sectional view of a cable sheath taken along the line D-D illustrated in FIG. 33. The following describes the configuration at the distal end side of the cable. The proximal end side (a universal cord side) also may have a similar configuration.

As illustrated in FIGS. 33 and 34, a cable 217 includes a comprehensive shield 217b that is a metal braid that coats a part except for the distal end side, of a coaxial cable bundle 217a that contains one or a plurality of signal cables that extend from the ultrasound transducer 27, a cable sheath 217c that coats the comprehensive shield 217b over an approximately entire length, except for a part of the proximal end, a first coated portion 217d that coats a part at the distal end side of the coaxial cable bundle 217a and including a border between the coaxial cable bundle 217a and the comprehensive shield 217b, a second coated portion 217e that coats the coaxial cable bundle 217a and the distal end side of the first coated portion 217d, a protective member 217f that includes the border between the coaxial cable bundle 217a and the comprehensive shield 217b in the radial direction and coats the cable sheath 217c, the first coated portion 217d, the second coated portion 217e, and at least a part of a fourth coated portion 217h described later, a third coated portion 217g that coats a part except for both ends, of the protective member 217f, and the fourth coated portion 217h that coats the cable sheath 217c.

The cable sheath 217c is provided over a whole circumference and the approximately entire length of the comprehensive shield 217b. The cable sheath 217c is formed with a material that fulfills withstand voltage performance, or formed so as to have a thickness that fulfills the withstand voltage performance.

The first coated portion 217d, the second coated portion 217e, the third coated portion 217g, and the fourth coated portion 217h are formed using heat shrinkable tubes. The first coated portion 217d prevents damage of the coaxial cable bundle 217a and the second coated portion 217e due to unraveling of the metal braid at a part exposed from the cable sheath, of the comprehensive shield 217b. The second coated portion 217e electrically insulates the distal end side of the coaxial cable bundle 217a. A comprehensive cable coupled to the first connector portion 251 has a configuration identical to that of the first coated portion 217d.

The protective member 217f, which is formed using polyimide or the like, is provided over a half circumference of the cable 217. When the third coated portion 217g is removed, in a case where the third coated portion 217g is cut using an edged tool, the protective member 217f prevents damage of the cable sheath 217c, the first coated portion 217d, the second coated portion 217e, and the fourth coated portion 217h. The disposition only over the half circumference restrains the cable 217 from increasing a diameter.

The third coated portion 217g, which is formed with a heat shrinkable tube, forms an outer-most coat of the cable 217. For this heat shrinkable tube, a material that fulfills the withstand voltage performance or a material having a thickness that fulfills the withstand voltage performance is employed. The third coated portion 217g electrically insulates the cable 217 at this coated part.

The fourth coated portion 217h, which is formed using a heat shrinkable tube, is provided over the whole circumference and the approximately entire length of the cable sheath 217c. The heat shrinkable tube is preferred to be formed using a transparent material. The formation of the fourth coated portion 217h ensures preventing the damage of the cable sheath 217c.

Thus, the present invention includes various embodiments without departing from the technical scope disclosed in the claim.

### Industrial Applicability

As described above, the endoscope system according to the present invention is effective for easily removing the water-resistant cap from the electric connector portion of the endoscope whose inside is in the negative pressure state.

### Reference Signs List

1 ENDOSCOPE SYSTEM
2 ENDOSCOPE
3 WATER-RESISTANT CAP
21 INSERTED PORTION
22 OPERATING UNIT
23 UNIVERSAL CORD
24 CONNECTOR
31 CAP MAIN BODY
31a SIDEWALL PORTION
31b BOTTOM PORTION
31c APERTURE PORTION
31d RIB
32 FIRST PACKING
32a CONVEX PORTION
33 CAM MAIN BODY
33a LOCKING GROOVE
34 SECOND PACKING
241, 241a FIRST CONNECTOR PORTION
242 SECOND CONNECTOR PORTION
2411 COUPLING PORTION
2411a BASE PORTION
2411b AIRTIGHTNESS PORTION
2411c, 2411k REDUCED DIAMETER PORTION
2411d, 2411e, 2411f, 2411g, 2411h, 2411i, 2411j, 2411m CUTOUT PORTION
24111 EXPANDED DIAMETER PORTION
2412 PROTRUDING PORTION
2413 LOCKING PIN

## Claims

1. An endoscope system comprising:
an endoscope including an inserted portion to be inserted into an inside of a subject and a connector portion for electrically coupling to an external connector, the endoscope being configured to observe the inside of the subject; and
a water-resistant cap configured to be airtightly installed at the connector portion, wherein
the connector portion includes:
a base portion having a tubular shape;
a tubular sleeve portion extending from the base portion and having a diameter smaller than a diameter of the base portion; and
an airtightness release portion provided at an end portion of the sleeve portion and configured to release an airtight state between the connector portion and the water-resistant cap,
the water-resistant cap includes:
a tubular cap main body having a closed bottom at one side; and
an abutting portion provided inside the cap main body and configured to abut on the sleeve portion, and
when the water-resistant cap is installed at the connector portion, the endoscope system takes any state of a first state where the abutting portion abuts on the sleeve portion on a side of the base portion with respect to the airtightness release portion to hold an airtightness between the water-resistant cap and the connector portion, and a second state where the abutting portion abuts on or faces the sleeve portion including the airtightness release portion to make non-airtight between the water-resistant cap and the connector portion.

2. The endoscope system according to claim 1, wherein the sleeve portion includes:
an airtightness portion that extends from the base portion, has a tubular shape having a diameter smaller than the diameter of the base portion, and is configured to be airtightly coupled to the abutting portion; and
a reduced diameter portion that has a tubular shape that extends from an end portion of the airtightness portion, the end portion of the airtightness portion being at a side different from the side of the base portion, the reduced diameter portion having an outer peripheral surface with a diameter decreasing toward a distal end, and
the airtightness release portion is provided at the reduced diameter portion.

3. The endoscope system according to claim 2, wherein
the airtightness release portion has a cutout shape where the sleeve portion is cut out from a distal end toward a proximal end.

4. The endoscope system according to claim 3, wherein
the airtightness release portion has a chamfered end portion continuous with the distal end of the sleeve portion.

5. The endoscope system according to claim 2, wherein
the airtightness release portion has a hole.

6. The endoscope system according to claim 2, wherein
the airtightness release portion has a concave shape where a part of the outer peripheral surface is depressed to an inner peripheral side.

7. The endoscope system according to claim 6, wherein
the airtightness release portion is formed such that a part of the outer peripheral surface is circumferentially depressed to the inner peripheral side.
